# FASCICULE DE BREVET EUROPEEN

(11) **EP 0 865 290 B1**
(45) Date de publication et mention de la délivrance du brevet: **03.03.2004**
(21) Numéro de dépôt: 96941107.3
(22) Date de dépôt: 04.12.1996
(51) Int. Cl.: A61K 35/78

(54) **COMPOSITIONS PHARMACEUTIQUES POUR APPLICATION BUCCALE COMPRENANT UN NSAID ET DES CERAMIDES**
BUKKALES ARZNEIMITTEL, WELCHES EIN NSAID UND CERAMIDE ENTHÄLT
PHARMACEUTICAL COMPOSITIONS FOR ORAL USE INCLUDING AN NSAID AND CERAMIDES

(30) Priorité: 06.12.1995 FR 9514392
(43) Date de publication de la demande: 23.09.1998
(73) Titulaire: SANOFI-SYNTHELABO, 75013 Paris (FR)
(72) Inventeur: FINIDORI, Claudine, F-92120 Montrouge (FR)
(74) Mandataire: Thouret-Lemaitre, Elisabeth
(86) Numéro de dépôt international: PCT/FR1996/001925
(87) Numéro de publication internationale: WO 1997/020572

(56) Documents cités:
- WO-A-93/09805

## Description

La présente invention a pour objet des compositions pharmaceutiques sous forme de dentifrice, bain de bouche ou gel pour application buccale comprenant un agent anti-inflammatoire non stéroïdien (AINS) et leur utilisation pour la prévention et le traitement des maladies parodontales.

Les AINS sont peu utilisés pour la prévention et le traitement des maladies parodontales, c'est à dire des maladies affectant le parodonte, telles que gingivites ou parodontites. Le parodonte consiste en les tissus de soutien de la dent. Il est essentiellement constitué de la gencive, de ligament, de l'os alvéolaire et du cément.
L'utilisation limitée des AINS pour la prévention et le traitement des maladies parodontales, peut s'expliquer par le fait que les compositions pour application buccale comprenant des AINS sont, généralement, insuffisamment efficaces. En effet, pour une bonne efficacité de ce type de compositions, une dose relativement élevée d'AINS est requise. Or de telles doses d'AINS sont généralement mal tolérées par le parodonte.

La présente invention propose alors de nouvelles compositions pour application buccales, comprenant des AINS, présentant une efficacité sensiblement améliorée, tout en étant bien tolérées par le parodonte. Ces compositions sont utiles pour la prévention et le traitement des maladies parodontales.

L'invention consiste ainsi en une composition pharmaceutique sous forme de dentifrice, bain de bouche ou gel pour application buccale, caractérisée en ce qu'elle comprend un agent anti-inflammatoire non stéroïdien (AINS) et des céramides.

La demanderesse a pu constater que les céramides permettent la vectorisation des AINS, c'est à dire qu'ils autorisent une amélioration de la disponibilité des AINS au niveau du site d'action. Les céramides forment ainsi, seuls ou associés avec d'autres composés décrits ci-après, une base vectorisante.

Les céramides sont des lipides comprenant généralement de 14 à 34 atomes de carbone, plus généralement de 17 à 23 atomes de carbone. Ils sont constitutifs des membranes plasmiques des cellules animales. Ils sont également présents dans les végétaux sous forme de mélanges lipidiques comprenant, outre les céramides, notamment des phospholipides tels les lécithines.
On connaît également des dérivés de céramides comprenant un ou plusieurs oses fixés sur la chaîne carbonée constitutive des céramides. Ces oses peuvent consister en le glucose, le galactose, le mannose, le fucose, la glucosamine ou la galactosamine. Ces dérivés de céramides comprenant un ou plusieurs oses font partie intégrante de la présente invention. Par souci de simplicité, et sauf indication contraire, on entend dans le cadre de la présente invention par le terme céramide, aussi bien des céramides per se que lesdits dérivés de céramides.

comme indiqué plus haut, les céramides peuvent être utilisés seuls ou sous forme de mélanges pouvant notamment comprendre, outre les céramides, d'autres lipides tels des phospholipides ainsi que, le cas échéant, des protéines.
De tels mélanges pouvant être utilisés dans le cadre de la présente invention, ainsi que leur procédé de préparation, sont décrits dans la demande internationale PCT/FR92/01048.

Les céramides de l'invention sont de préférence d'origine végétale.

Une composition pharmaceutique selon l'invention peut comprendre de 0,005 à 5 % en poids, de préférence de 0,2 à 2 % en poids de céramides.

Les céramides mis en oeuvre peuvent se présenter sous forme d'une émulsion aqueuse dont la phase huile est constituée, au moins pour partie, de céramides et, le cas échéant, d'autres lipides.

L'AINS est avantageusement choisi parmi la benzydamine, le kétoprofène ou le lysinate de kétoprofène. Une composition pharmaceutique selon l'invention peut comprendre de 0,005 à 10% en poids, de préférence de 0,25 à 2,5% en poids d'AINS.

Une composition pharmaceutique pour application buccale selon l'invention se présente sous forme de dentifrices, de bains de bouche ou de gels. Outre l'AINS et les céramides, la composition pharmaceutique peut comprendre au moins un autre excipient tel que polymères d'acide acrylique, polymères carboxyvinyliques, silices, propylèneglycol, polyéthylèneglycols, esters d'acides gras, paraffine, éthanol, isopropanol, diéthanolamine, triéthanolamine, glycérol, hydroxyéthylcellulose et carboxyméthylcellulose (CMC), pour les gels ; carbonate ou phosphate de calcium, silices ou alumines hydratées, carboxyméthylcellulose, carraghénates, alginates, laurylsulfate ou laurylsarcosinate de sodium, dodécanediol polyglycérolé, glycérol, sorbitol et propylèneglycol, pour les dentifrices ; alcools tels que glycérol et éthanol, pour les bains de bouche.

Ces compositions pharmaceutiques peuvent également comprendre, selon le cas et de manière classique, des agents conservateurs, des émulsifiants, des sels tampons, des édulcorants, des arômes et des colorants.

Les compositions pharmaceutiques selon l'invention peuvent être préparées de manière conventionnelle pour l'homme du métier.

Comme démontré dans les exemples 1 à 3 ci-après, les céramides présentent d'une part, une activité inhibitrice de l'enzyme leucocytaire humaine, enzyme responsable de la destruction du tissu conjonctif au cours de réactions inflammatoires et d'autre part, induisent un effet anti-inflammatoire supérieur à celui de l'AINS utilisé seul à la même dose. Les compositions pharmaceutiques de la présente invention peuvent donc être utilisées dans la prévention et le traitement des maladies parodontales telles que gingivites ou parodontites.

Les exemples suivants illustrent l'invention. Ils ont tous été réalisés avec des céramides végétales extraites de farine de blé, commercialisées par les Laboratoires INOCOSM. Ces céramides végétales contenaient 80 % de glycosylcéramides ainsi que 10 % de gliadine et 10 % de lipides apolaires.

### Exemple 1 :

On a recherché le rôle des céramides dans la prévention de la destruction de tissus conjonctifs qui se produit en particulier dans les maladies parodontales.
Les maladies parodontales sont caractérisées par l'inflammation gingivale.
Or, au cours de processus inflammatoires, les enzymes protéolytiques libérés peuvent, s'ils ne sont pas inactivés par des inhibiteurs de protéase, entraîner des destructions tissulaires.
En particulier, le rôle de l'élastase dans la dégradation de la matrice extra-cellulaire gingivale lors d'une inflammation a été démontré.

Des essais ont donc été réalisés in vitro et ex vivo, pour rechercher les effets des céramides sur l'élastase leucocytaire.
L'essai in vitro a consisté à incuber l'élastase leucocytaire humaine avec des céramides végétales pendant 5 min, temps nécessaire à la formation du complexe enzyme-inhibiteur, puis avec un substrat synthétique, le N-méthoxysuccinyl-Ala-Ala-Pro-Val-p-nitroanilide.
L'enzyme provoque l'hydrolyse du substrat synthétique en un produit coloré, la p-nitroaniline, dont l'apparition est suivie par son absorbance à 410 nm. La vitesse d'apparition du produit de la réaction est mesurée en présence ou en l'absence de céramides végétales et avec différentes concentrations de substrat et de céramides. Le rapport entre les vitesses initiales permet de déterminer le pourcentage d'inhibition de l'enzyme en fonction de la concentration en céramides dans le milieu réactionnel.

Les résultats obtenus ont montré que les céramides sont des inhibiteurs de l'élastase leucocytaire et que la concentration en céramides produisant une inhibition de 50 % est de 32,7 µg/ml.

L'étude ex vivo a été réalisée sur des biopsies cutanées humaines et des biopsies gingivales de rats. Les gencives de rats et les gencives humaines présentent en effet une structure comparable à celle de la peau, comportant des fibres préélastiques dans la partie superficielle de la matrice extra cellulaire et des fibres élastiques matures en profondeur, les fibres de collagènes étant le constituant majoritaire du tissu conjonctif.
Les biopsies, en coupes de 8 mm d'épaisseur, ont été réparties en 4 groupes soumis aux traitements suivants :
traitement 1 : incubation dans 200 µl de tampon Tris-HCl, à pH = 8, pendant 12 heures à 37°C (groupe témoin négatif).
traitement 2 : incubation avec l'élastase leucocytaire seule pendant 12 heures à 37°C (groupe témoin).
traitement 3 : action de céramides végétales seules (200 µl d'une solution à 1 %), puis 2 lavages au PBS et enfin incubation avec l'élastase leucocytaire humaine (200 µl d'une solution à 10 µg/ml pour la peau et à 2 µg/ml pour les gencives). Ce traitement permet l'étude de la formation d'un complexe substrat-céramide.
traitement 4 : mélange de céramides végétales avec l'élastase leucocytaire humaine pendant 30 mn, puis incubation du mélange avec les coupes, à 37°C pendant 12 heures. Ce traitement permet l'étude de la formation d'un complexe enzyme-céramide.
Les coupes sont ensuite rincées, fixées à l'alcool à 70 % et colorées soit à la catéchine-fuschine pour visualiser les fibres élastiques, soit au rouge Sirius pour visualiser les fibres de collagènes.

La détermination, par analyse d'images informatisée, de la fraction volumétrique occupée par les fibres permet de mesurer le pourcentage d'inhibition de l'enzyme.

Après le traitement 2, on a observé une destruction importante des fibres élastiques matures par l'élastase leucocytaire.

Les résultats obtenus après le traitement 4 ont montré une protection des fibres élastiques de plus de 90 %, due à l'action inhibitrice des céramides végétales sur l'enzyme.

On a également observé une protection des fibres de collagène après les traitements 3 et 4, ce qui indique une interaction des céramides à la fois avec les fibres de collagènes et avec l'élastase leucocytaire humaine.

### Exemple 2 :

On a étudié l'effet anti-inflammatoire des compositions de l'invention par détermination de leur pouvoir antiradicalaire vis-à-vis de l'anion super oxyde in vitro.

Il est connu, en effet, que les polynucléaires activés au cours du processus anti-inflammatoire produisent des radicaux libres.
On sait également que l'inflammation s'accompagne de l'activation d'enzymes comme la cyclooxygénase, qui activent la formation radicalaire.
L'action antiradicalaire d'une composition anti-inflammatoire est donc un bon indice de son pouvoir anti-inflammatoire.

La détermination du pouvoir antiradicalaire de compositions selon l'invention a été réalisée par mesure de la diminution de la vitesse de réduction du Cytochrome C lors de l'addition au milieu réactionnel du produit à étudier.
L'anion superoxyde est généré par l'action de la xanthine oxydase sur la xanthine et entraîne, en l'absence d'une molécule capable de le capter, la réduction du cytochrome C. L'apparition du Cytochrome C réduit est suivie au spectrophotomètre, à 550 nm.

Pour réaliser ces essais, des émulsions comprenant un AINS et des céramides ont été préparées.

Ces émulsions, dont la composition était la suivante :

| | |
|---|---|
| céramides végétales | 0,5 g |
| lécithine | 0,5 g |
| gomme xanthane | 0,3 g |
| AINS | 0,5 g |
| eau qsp | 100,0 g |

ont été préparées par addition, sous vive agitation, des céramides, de la lécithine et de l'AINS, puis refroidissement sous agitation et passage à l'autoclave.
Elles ont ensuite été diluées au 1/2 et au 1/4 pour obtenir des concentrations de 2,5 mg/ml et 1,25 mg/ml en AINS.

De telles compositions contenant respectivement du kétoprofène, du lysinate de kétoprofène et de la benzydamine ont été testées et les résultats comparés aux résultats obtenus avec des solutions contenant les mêmes constituants à l'exception des céramides.

Les résultats obtenus ont montré que la protection antiradicalaire des compositions comprenant des céramides augmentait de 6 à 22 % par rapport aux compositions ne comprenant pas de céramides, selon l'AINS et sa concentration.

### Exemple 3 :

Des essais d'activité anti-inflammatoire ont également été réalisés in vivo.
Ces essais consistent à provoquer une inflammation de la patte chez des rats adultes et à suivre l'évolution de l'oedème par mesure du volume de la patte.

Les conditions expérimentales étaient les suivantes : des rats, d'un poids de 200 à 220 g, ont été traités par gavage, 18 heures et 1 heure avant l'administration de l'agent inflammatoire, par le produit AINS à étudier, associé ou non à des céramides, administré sous un volume de 0,5 ml.
Des groupes témoins ont reçu une solution de chlorure de sodium à 0,9%.

La réaction inflammatoire est provoquée par injection sous-cutanée de 0,1 ml d'une suspension de carraghénine dans du sérum physiologique. Le volume de la patte est mesuré à l'aide d'un pléthismographe à mercure, avant le début de l'inflammation, une demi-heure après, puis toutes les heures jusqu'à la sixième heure et enfin 24 heures après. Les variations de volume sont mesurées en cm sur une échelle graduée.
Les essais ont été faits avec le lysinate de kétoprofène à 1 et 3 mg/kg, associé ou non à 0,5 % de céramides, et le kétoprofène, à 1 mg/kg, associé ou non à 1 % de céramides.

Les résultats obtenus ont montré que le lysinate de kétoprofène à 1 et 3 mg/kg, associé à des céramides, induit un effet anti-inflammatoire significatif par rapport au témoin, à partir du temps 3 heures, effet qui persiste jusqu'à 6 heures.

Le lysinate de kétoprofène à 1 mg/kg, associé à des céramides, a un effet anti-inflammatoire significativement supérieur à celui du même composé à 1 et 3 mg/kg, non associé à des céramides (réduction d'environ 60 % par rapport au groupe traité par le composé à 1 et 3 mg/kg, non associé à des céramides, au temps 4 heures).

Le kétoprofène à 1 mg/kg, associé ou non à des céramides, induit un effet anti-inflammatoire significatif par rapport au témoin, à partir de 4 heures, cet effet persistant jusqu'à 5 heures.

A 1 mg/kg, le kétoprofène associé à des céramides a un effet supérieur à celui du kétoprofène non associé à des céramides à la même dose (réduction d'environ 15 % par rapport au groupe traité par le composé non associé à des céramides, au temps 5 heures).

Dans les exemples qui suivent les % sont exprimés en poids.

### Exemple 4 : dentifrice

| | |
|---|---|
| kétoprofène | 2,00 g |
| céramides | 2,00 g |
| fluorure (NaF ou KF) | 1000 à 5000 ppm |
| silices | 18,00 g |
| sorbitol 70 % | 25,00 g |
| carboxyméthylcellulose | 1,50 g |
| laurylsulfate de sodium | 0,75 g |
| dodécanediol polyglycérolé | 0,75 g |
| parabens* | 0,30 g |
| arôme menthe | 1,20 g |
| eau qsp | 100,00 g |

| | |
|---|---|
| * mélange 50/50 (poids/poids) de para-hydroxybenzoate de méthyle et de para-hydroxybenzoate de propyle. | |

### Exemple 5 : dentifrice

| | |
|---|---|
| benzydamine | 1,00 g |
| céramides | 0,50 g |
| silices | 15,00 g |
| glycérol | 20,00 g |
| carraghénate de sodium | 2,00 g |
| laurylsulfate de sodium | 1,50 g |
| parabens* | 0,30 g |
| arôme menthe | 1,20 g |
| eau qsp | 100,00 g |

| | |
|---|---|
| mélange 50/50 (poids/poids) de para-hydroxybenzoate de méthyle et de para-hydroxybenzoate de propyle. | |

### Exemple 6 : bain de bouche

| | |
|---|---|
| kétoprofène | 1,00 g |
| céramides | 1,50 g |
| alcool | 5,00 g |
| colorant | 0,30 g |
| arôme menthe | 0,20 g |
| saccharinate de sodium | 0,20 g |
| eau qsp | 100,00 ml |

### Exemple 7 : bain de bouche

| | |
|---|---|
| benzydamine | 0,50 g |
| céramides | 0,50 g |
| glycérol | 2,00 g |
| alcool | 5,00 g |
| Tween 80 | 0,50 g |
| saccharinate de sodium | 0,15 g |
| arôme | 0,12 g |
| eau qsp | 100,00 ml |

### Exemple 8 : gel buccal

| | |
|---|---|
| kétoprofène | 3,00 g |
| céramides | 2,00 g |
| glycérol | 20,00 g |
| hydroxyéthylcellulose | 5,00 g |
| arôme | 0,30 g |
| saccharinate de sodium | 0,30 g |
| eau qsp | 100,00 g |

### Exemple 9 : gel buccal

| | |
|---|---|
| benzydamine | 2,00 g |
| céramides | 1,50 g |
| alcool à 95° | 40,00 g |
| glycérol | 10,00 g |
| carboxyméthylcellulose | 5,00 g |
| arôme | 0,30 g |
| eau qsp | 100,00 g |

## Revendications

1. Composition pharmaceutique sous forme de dentifrice, bain de bouche ou gel pour application buccale, **caractérisée en ce qu'**elle comprend un agent anti-inflammatoire non stéroïdien (AINS) et des céramides.

2. Composition selon la revendication 1, **caractérisée en ce que** l'agent AINS est la benzydamine, le kétoprofène ou le lysinate de kétoprofène.

3. Composition selon l'une quelconque des revendications 1 ou 2, **caractérisée en ce que** les céramides se présentent sous forme d'une émulsion aqueuse.

4. Composition selon l'une quelconque des revendications 1 à 3, **caractérisée en ce qu'**elle comprend de 0,005 à 5 % en poids de céramides.

5. Composition selon l'une quelconque des revendications 1 à 4, **caractérisée en ce que** les céramides sont d'origine végétales.

6. Composition selon l'une quelconque des revendications 1 à 5, **caractérisée en ce qu'**elle contient 0,005 à 10 % en poids d'agent AINS.

7. Utilisation d'une composition selon l'une quelconque des revendications 1 à 6 pour la préparation d'un médicament destiné à la prévention et au traitement des maladies parodontales telles que gingivites et parodontites.

## Claims

1. Pharmaceutical composition in the form of a dentifrice, a mouthwash or a gel for buccal use, **characterized in that** it includes a nonsteroidal antiinflammatory (NSAI) agent and ceramides.

2. Composition according to Claim 1, **characterized in that** the NSAI agent is benzydamine, ketoprofen or ketoprofen lysinate.

3. Composition according to either one of Claims 1 and 2, **characterized in that** the ceramides are in the form of an aqueous emulsion.

4. Composition according to any one of Claims 1 to 3, **characterized in that** it includes from 0.005 to 5% by weight of ceramides.

5. Composition according to any one of Claims 1 to 4, **characterized in that** the ceramides are of plant origin.

6. Composition according to any one of Claims 1 to 5, **characterized in that** it includes from 0.005 to 10% by weight of NSAI agent.

7. Use of a composition according to any one of Claims 1 to 6 for preparing a medicament which is intended for preventing and treating periodontal disorders such as gingivites and periodontites.

## Patentansprüche

1. Pharmazeutische Zubereitung in Form eines Zahnputzmittels, einer Mundspülung oder eines Gels, für die bukkale Anwendung, **dadurch gekennzeichnet, daß** sie ein nicht-steroidales antiinflammatonsches Mittel (AINS) und Ceramide enthält.

2. Zubereitung nach Anspruch 1, **dadurch gekennzeichnet, daß** das AINS-Mittel Benzydamin, Ketoprofen oder Ketoprofen-lysinat ist.

3. Zubereitung nach einem der Ansprüche 1 oder 2, **dadurch gekennzeichnet, daß** die Ceramide in Form einer wäßrigen Emulsion vorliegen.

4. Zubereitung nach einem der Ansprüche 1 bis 3, **dadurch gekennzeichnet, daß** sie 0,005 bis 5 Gew.-% Ceramide enthält.

5. Zubereitung nach einem der Ansprüche 1 bis 4, **dadurch gekennzeichnet, daß** die Ceramide pflanzlichen Ursprungs sind.

6. Zubereitung nach einem der Ansprüche 1 bis 5, **dadurch gekennzeichnet, daß** sie 0.005 bis 10 Gew.-% des AINS-Mittels enthält.

7. Verwendung einer Zubereitung nach einem der Ansprüche 1 bis 6 für die Herstellung eines Arzneimittels zur Vorbeugung und zur Behandlung von parodontalen Erkrankungen, wie Gingivitis und Parodontitis.
